(19) Europäisches Patentamt / European Patent Office / Office européen des brevets

(11) **EP 4 256 358 B1**

(12) **FASCICULE DE BREVET EUROPEEN**

(45) Date de publication et mention de la délivrance du brevet:
**05.02.2025 Bulletin 2025/06**

(21) Numéro de dépôt: **21848262.8**

(22) Date de dépôt: **02.12.2021**

(51) Classification Internationale des Brevets (IPC):
**G01R 33/32** *(2006.01)*    **G01R 33/44** *(2006.01)*
**G01R 33/34** *(2006.01)*

(52) Classification Coopérative des Brevets (CPC):
**G01R 33/326**; G01R 33/34061; G01R 33/34069;
G01R 33/445

(86) Numéro de dépôt international:
**PCT/FR2021/052187**

(87) Numéro de publication internationale:
**WO 2022/117969 (09.06.2022 Gazette 2022/23)**

(54) **SYSTÈME DE DÉTECTION ET D'ACQUISITION RF BAS-BRUIT À BASE DE SQUID ET ÉQUIPEMENTS INTÉGRANT CE SYSTÈME**

RAUSCHARMES SQUID-BASIERTES RF-DETEKTIONS- UND ERFASSUNGSSYSTEM UND AUSRÜSTUNG MIT DIESEM SYSTEM

LOW-NOISE RF DETECTION AND ACQUISITION SQUID-BASED SYSTEM AND EQUIPMENT INCLUDING THIS SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **03.12.2020 FR 2012642**

(43) Date de publication de la demande:
**11.10.2023 Bulletin 2023/41**

(73) Titulaire: **Chipiron**
**75169 Paris Cedex 19 (FR)**

(72) Inventeur: **LABAT, Dimitri**
**75020 Paris (FR)**

(74) Mandataire: **IP Trust**
**2, rue de Clichy**
**75009 Paris (FR)**

(56) Documents cités:
**WO-A1-2006/052236    US-A1- 2013 271 142**

• **CHEN HSIN-HSIEN ET AL: "A compact SQUID-detected magnetic resonance imaging system under microtesla field in a magnetically unshielded environment", JOURNAL OF APPLIED PHYSICS, AMERICAN INSTITUTE OF PHYSICS, US, vol. 110, no. 9, 1 November 2011 (2011-11-01), pages 93903 - 93903, XP012154562, ISSN: 0021-8979, [retrieved on 20111101], DOI: 10.1063/1.3656984**
• **PANU T. VESANEN ET AL: "Hybrid ultra-low-field MRI and magnetoencephalography system based on a commercial whole-head neuromagnetometer", MAGNETIC RESONANCE IN MEDICINE, vol. 69, no. 6, 1 June 2013 (2013-06-01), pages 1795 - 1804, XP055358397, ISSN: 0740-3194, DOI: 10.1002/mrm.24413**
• **DEMACHI KAZUMA ET AL: "T1-Weighted Image by Ultra-Low Field SQUID-MRI", IEEE TRANSACTIONS ON APPLIED SUPERCONDUCTIVITY, IEEE SERVICE CENTER, LOS ALAMITOS, CA, US, vol. 29, no. 5, 4 March 2019 (2019-03-04), pages 1 - 5, XP011719476, ISSN: 1051-8223, [retrieved on 20190409], DOI: 10.1109/TASC.2019.2902772**

EP 4 256 358 B1

**Description**

DOMAINE DE L'INVENTION

La présente invention concerne un système de détection et d'acquisition RF bas-bruit à base de SQUID. Elle vise également des équipements intégrant ce système, notamment un équipement d'imagerie par résonance magnétique.

ETAT DE LA TECHNIQUE

Il est bien connu d'utiliser des systèmes de détection à base de SQUID (pour « Superconducting QUantum Interférence Device »: *dispositif d'interférence quantique supraconducteur*) dans des équipements d'imagerie par résonance magnétique (IRM).

Lors d'une expérience d'IRM, le sujet et placé dans un champ magnétique stationnaire et homogène $B_0$. On soumet alors l'échantillon à un signal RF de fréquence $\omega$ accordée à la fréquence de Larmor $\omega_0 = \gamma B_0$ des protons dans le champ $B_0$, avec $\gamma/(2\pi) = 42, 6$ MHz.T$^{-1}$ le rapport gyromagnétique du proton. A la suite de cette excitation, l'échantillon émet un signal de fréquence $\omega_0$ capté par un système de détection, dont le principe dans les équipements commerciaux modernes est basé sur une antenne inductive refroidie.

Le paradigme habituel de l'IRM est de choisir le champ magnétique le plus élevé possible, pour deux raisons :

- La première est qu'un champ plus élevé permet de polariser un plus grand nombre de protons, ce qui conduit à plus de signal, donc un temps d'expérience réduit et une résolution d'image plus fine.
- La deuxième est que la détection RF classique se fait avec une antenne inductive, dont la sensibilité est proportionnelle à $\omega$.

En tenant compte du niveau de bruit thermique en $\omega^{1/4}$, le seul de détection de l'antenne Faraday suit une loi en $\omega^{-3/4}$. Puisque $\omega$ est proportionnel au champ de travail $B_0$, travailler à haut champ permet d'abaisser le seuil de détectivité. Un équipement IRM clinique typique travaille à 1, 5 ou 3 T. Certains modèles montent à 7 T, et des expériences visent à atteindre 11, 7 T.

Toutefois augmenter le champ produit du bruit ionique dans l'échantillon, dégrade la qualité d'image en raison de différence de susceptibilité magnétique des tissus, et a tendance à niveler les contrastes, sans compter les contraintes habituelles liées aux hauts champs : bobines supraconductrices coûteuses à produire et entretenir, salles IRM blindées magnétiquement, pas d'IRM pour les porteurs de stimulateurs cardiaques ou pacemakers, ni pour les soldats blessés par balle.

Le document CN105137374A divulgue un appareil IRM avec détection SQUID en résolution nanométrique, mettant en oeuvre un procédé d'imagerie par résonance magnétique et un dispositif à ultra-haute résolution. Ce procédé comprend au moins une étape de placement d'un échantillon testé dans la plage de travail d'une source de gradient de champ magnétique et d'un dispositif d'interférence quantique nano supraconducteur, une étape d'utilisation d'une source de champ magnétique statique pour appliquer un champ magnétique statique à l'échantillon testé, et utiliser une source de radiofréquence pour appliquer une impulsion de radiofréquence de résonance magnétique nucléaire à l'échantillon testé pour exciter l'échantillon testé afin de générer une résonance magnétique nucléaire, une étape d'utilisation du dispositif d'interférence quantique nano supraconducteur pour coupler directement l'échantillon testé pour détecter le magnétisme nucléaire signal de spectre de résonance généré par l'échantillon testé, et une étape d'établissement de l'image de l'échantillon testé en fonction du signal de spectre de résonance magnétique nucléaire détecté et des informations de distribution spatiale de la source de gradient de champ magnétique. Le dispositif d'interférence quantique nano supraconducteur est utilisé comme détecteur, l'imagerie par résonance magnétique avec une résolution de niveau nanométrique peut être réalisée, la mesure n'est pas perturbée par des vibrations et un signal de champ électrique, l'échantillon peut être directement couplé avec le détecteur à une distance proche, la plage d'image est augmentée, et le travail dans un champ magnétique fort est réalisé.

Le document JP2010256318A divulgue un équipement de résonance magnétique nucléaire protonique haute résolution et l'imagerie (RMN / IRM) utilisant un magnétomètre à dispositif d'interférence quantique supraconducteur (SQUID) à température critique élevée (haute Tc) via un transformateur de flux magnétique dans un champ magnétique micro Tesla. L'invention concerne un procédé et un appareil. Le SQUID et la bobine d'entrée sont installés dans une cuve supraconductrice qui protège le bruit ambiant et met le SQUID dans un état de fonctionnement stable. Le signal RMN peut être maintenu même lorsque l'échantillon est éloigné du détecteur SQUID.

Le document CN1287160C divulgue un appareil RMN et IRM à détection SQUID, avec prépolarisation, dans lequel les signaux de résonance magnétique nucléaire (RMN) sont détectés dans des champs microtesla. La prépolarisation dans des champs millitesla est suivie d'une détection avec un magnétomètre à dispositif d'interférence quantique supraconducteur à courant continu non accordé (SQUID).

Le document WO2006052236A1 divulgue un appareil RMN et IRM à détection SQUID, avec prépolarisation et antenne primaire Faraday. L'imagerie par résonance magnétique dans un champ magnétique très faible est basée sur les RMN de champs très faibles. Des champs magnétiques de gradients sont appliqués, et des images sont construites à partir des signaux RMN détectés.

Ces dispositifs de détection et d'acquisition à base de SQUID présentent l'inconvénient de nécessiter des méthodes de prépolarisation et mettent en oeuvre des SQUIDS haut-Tc.

Le document Chen Hsin-Hsien et al: "A compact SQUID-detected magnetic resonance imaging system under microtesla field in a magnetically unshielded environment", Journal of Applied Physics, American Institute of Physics, vol. 110, no. 9, 1 novembre 2011 (2011-11-01),, divulgue un système de détection et d'acquisition radiofréquence à base de SQUID, prévu notamment pour être intégré dans un appareil à résonance magnétique nucléaire, ce système de détection comprenant une antenne primaire de détection, un transformateur de flux ayant un enroulement primaire relié à l'antenne primaire de détection, un dispositif SQUID, un dispositif de cryogénie prévu pour refroidir le dispositif SQUID et le transformateur de flux, un étage de traitement du signal de détection secondaire émis par le dispositif SQUID, cet étage de traitement comprenant une boucle à verrouillage de flux et étant prévu pour délivrer un signal analogique d'acquisition. Le document US2013271142A1 divulgue des dispositifs, des composants et des procédés d'IRM SQUID à faible champ. Ils comprennent un instrument d'IRM portable à faible champ (SQUID) et un système d'IRM portable à faible champ à base de SQUID à utiliser sous un lit. Cet instrument d'IRM met en oeuvre un gradiomètre supraconducteur du second ordre adapté pour être utilisé avec un dispositif IRM à faible champ SQUID.

Le but de l'invention est de proposer un système de détection et d'acquisition RF bas-bruit à base de SQUID qui soit de conception plus simple et moins coûteuse que les systèmes précités de l'art antérieur, et qui en améliore les performances en particulier en termes de ratio signal à bruit.

EXPOSE DE L'INVENTION

Cet objectif est atteint avec un système de détection et d'acquisition radiofréquence (RF) à base de SQUID, prévu notamment pour être intégré dans un appareil à résonance magnétique nucléaire (IRM ou RMN), comprenant:

- une antenne primaire de détection de type volumique,
- un transformateur de flux ayant un enroulement primaire relié à l'antenne primaire de détection,
- un dispositif SQUID, agencé pour capter le flux magnétique capturé par l'antenne primaire et reproduit par un enroulement d'entrée au sein dudit dispositif SQUID via le transformateur de flux, et délivrer un signal de détection secondaire,
- un dispositif de cryogénie prévu pour refroidir le dispositif SQUID,
- un étage de traitement du signal de détection secondaire émis par le dispositif SQUID, pour délivrer un signal analogique d'acquisition, comprenant une boucle à verrouillage de flux (FLL) prévue pour linéariser la réponse du dispositif SQUID.

Suivant l'invention, le dispositif SQUID est de type à basse température critique, le dispositif de cryogénie est en outre prévu pour refroidir le transformateur de flux, et l'antenne primaire de détection comprend des bobines de Helmholtz ou en selles de cheval. Ces antennes volumiques peuvent être accordées au signal à détecter, ou non-accordées.

Travailler à bas champ magnétique, autour de 1mT ou moins, permet par ailleurs de bénéficier d'une augmentation drastique des contrastes T1, ouvrant la voie à des possibilités d'imagerie uniques au régime bas champ. Ce fait est détaillé dans la publication « SQUID-detected MRI at 132 µT with Ti-weighted contrast established at 10 µT-300 mT » de S.K. Lee et al, paru dans le Journal « Magnetic Résonance in Medicine » Volume 53-1, Janvier 2005, pages 9-14.

La boucle à verrouillage de flux (FLL) peut avantageusement comprendre un amplificateur bas-bruit (LNA) qui peut inclure un amplificateur à hétérostructure semiconductrice ou un système d'amplification à base de SQUID. Le système d'amplification à base de SQUID est présenté dans le document US2013271142A1 . Pour la mise en oeuvre de ces technologies dans le cadre de la présente invention, on pourra utilement se référer à l'ouvrage « The SQUID handbook : Fundamentals of Technology and Applications of SQUIDs and SQUID systems" par John Clarke, éditions Wiley-VCH 2004.

Le système de détection et d'acquisition selon l'invention peut en outre comprendre une ou plusieurs bobines de compensation active du bruit externe à ce système.

Le système de détection et d'acquisition selon l'invention peut en outre comprendre un ou plusieurs écrans de blindage passif du système. Ce blindage passif peut être de nature magnétique, notamment via des ferrites, du mu-métal, du Cryoperm®, du Metglas®, ou tous autres matériaux ou alliages de haute perméabilité magnétique. Ce blindage peut également être en métal, par exemple en cuivre ou en aluminium.

Dans une version préférée de l'invention, l'antenne primaire de détection coopère avec le transformateur de flux pour concentrer le flux capté par le dispositif SQUID.

On peut aussi prévoir que le système de détection et d'acquisition selon l'invention comprenne en outre au sein du transformateur de flux une bobine de contre-réaction d'inductance agencée pour réagir aux variations du flux entrant, de façon à maintenir le dispositif SQUID à son niveau de sensibilité en flux maximale.

Suivant un autre aspect de l'invention, il est proposé un équipement d'imagerie par résonance magnétique (IRM) comprenant un système (B) de détection et d'acquisition radiofréquence (RF) à base de SQUID selon l'invention, un dispositif porte-antenne (A), intégrant l'antenne primaire de détection de type volumique et relié audit système (B) de détection et d'acquisition, et un étage de conversion analogique-numérique (C), prévu pour convertir le signal analogique d'acquisition en données numériques adaptées pour un post-traitement en vue de générer et afficher une image IRM.

Cet équipement IRM peut par exemple être couplé à un dispositif de magnétoencéphalographie (MEG).

Le système de détection et d'acquisition RF à base de SQUID peut être mis en oeuvre dans un équipement de résonance magnétique nucléaire (RMN), ou dans un équipement capteur magnétique à base de SQUID pour la prospection de métaux, en vue de détecter une onde radiofréquence (RF) émise par une veine métallique en réponse à une émission d'une onde radiofréquence (RF) dans un sol.

On peut aussi prévoir un équipement capteur radiofréquence ultrasensible incluant un système de détection et d'acquisition selon l'invention, ou encore un équipement de radioastronomie opérant dans le domaine des radiofréquences (RF) incluant un système de détection et d'acquisition RF à base de SQUID selon l'invention.

## DESCRIPTION DES FIGURES

On comprend mieux l'invention en référence aux figures décrites ci-après :

- La Figure 1 est un schéma du circuit de détection RF et de traitement du signal à base de SQUID.

- La figure 2 est un schéma de principe d'une acquisition IRM à partir de notre invention de détection RF.

- La figure 3 illustre plusieurs géométries d'antenne primaire de détection.

## DESCRIPTION DETAILLEE

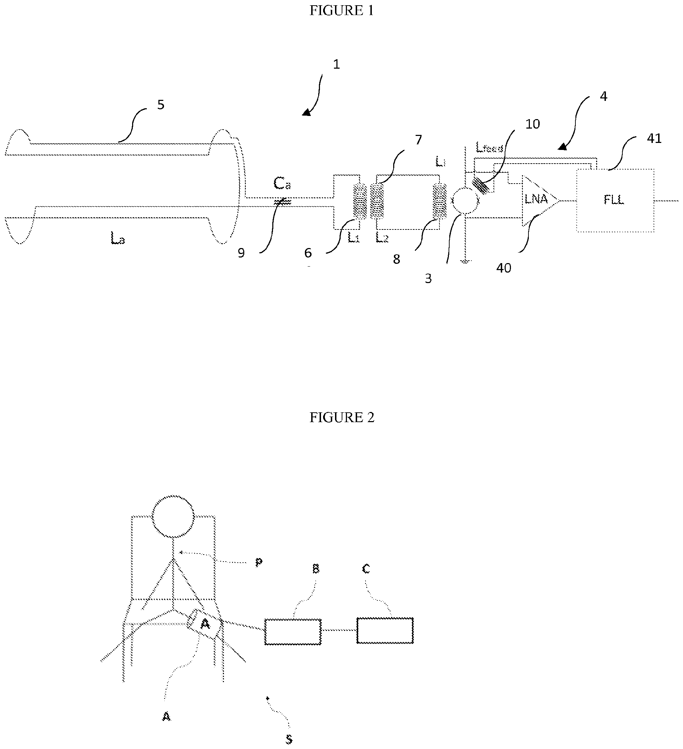

On va maintenant décrire, en référence à la figure 1, un exemple de réalisation d'un système 1 de détection et d'acquisition RF à base de SQUID selon l'invention.

Ce système 1 de détection et d'acquisition RF à base de SQUID comprend une antenne inductive primaire 5 de type volumique, réalisée sous la forme de bobines de Helmholtz ou en selles de cheval, ou de toute autre type volumique, notamment gradiométrique, qui est reliée , dans le cas où l'antenne est résonante, via un condensateur 9 de couplage de capacitance $C_a$ à un enroulement primaire 6, d'inductance $L_1$, d'un transformateur de flux 2 présentant un enroulement secondaire 7, d'inductance $L_2$, en série avec une bobine d'entrée 8 d'inductance Li générant un flux capté par un dispositif SQUID 3.

Si l'antenne n'est pas résonante, la capacité n'est pas présente et l'antenne 5 est directement reliée à l'enroulement primaire 6.

Le transformateur de flux 2 et le dispositif SQUID 3 sont maintenus à basse température dans un dispositif de cryogénie (non représenté) comprenant un *pulse tube* (tube pulsé) tel que le produit PT403 commercialisé par CryoMech®.

Un étage 4 de traitement du signal de détection secondaire comprend un pré-amplificateur 40 (LNA) de la tension mesurée aux bornes du dispositif SQUID 3. Cette mesure de tension représente un signal de détection qui est appliqué en entrée d'un circuit 41 de boucle à verrouillage de flux FLL incluant un amplificateur bas-bruit et relié à une bobine de contre-réaction 10 d'inductance $L_{feed}$ prévue pour réagir aux variations de flux entrant afin de maintenir le dispositif SQUID 3 à son niveau de sensibilité en flux maximal. Le procédé de la boucle à verrouillage de flux est divulgué dans le document US20120206136A1.

Un exemple de caractéristiques quantitatives du système 1 de détection et d'acquisition RF à base de SQUID est donné ci-dessous :

- Largeur temporelle typique du train d'onde à détecter : $T_2$*~ 50 ms
- Fréquence centrale de l'antenne primaire $\omega_0$ - 40 kHz
- Bande passante du primaire $\Delta\omega$ - 20 kHz
- Facteur de qualité du primaire $Q$ - 2 (si antenne résonante)
- Intensité du champ magnétique au niveau de l'antenne primaire $B_p$ , de l'ordre de la centaine de fT au pT
- Inductance de la bobine d'entrée du SQUID $L_i$ = 720 nH
- Inductance propre de l'antenne primaire $L_a$ - 0.1 mH

- Résistance de l'antenne primaire $R_a$ = 1Ω
- Capacité de résonance de l'antenne primaire $C_a$ = 6 μF

Antenne inductive

Au vu des applications visées, on choisit une géométrie de type volumique pour l'antenne 5. Des exemples de cette géométrie incluent les bobines de Helmholtz, les bobines de type « selle de cheval », ou d'autres géométries plus complexes, notamment des géométries gradiométriques. Cette géométrie permet de récolter un maximum de signal tout en permettant, par sa géométrie ouverte, un confort relatif pour le patient. L'antenne primaire Faraday 5 doit être accordée au signal IRM. Cette antenne 5 a pour inductance propre La, pour résistance $R_a$, que l'on cherchera à diminuer le plus possible, afin de minimiser le bruit de Johnson-Nyquist dans l'antenne.

Les paramètres $L_a$, $R_a$ sont fixés par la géométrie d'antenne choisie et le type de matériau constituant l'antenne. On a alors la possibilité de rendre l'antenne résonante, ce qui permet deux choses :

- Le facteur de qualité Q de l'antenne résonante permet d'amplifier naturellement le signal capté,

- la bande passante $\Delta\omega$ de l'antenne permet de filtrer les signaux captés et de rejeter le bruit électromagnétique hors de la bande d'intérêt $\Delta\omega$.

Cette implémentation fonctionne aussi bien pour une antenne résonante que pour une antenne non-résonante. Est exposé ci-dessous le cas de l'antenne résonante.

On règle la capacité de sorte que la fréquence propre de l'antenne $\omega_a = 1/\sqrt{LaCa}$ soit accordée sur la fréquence $\omega_0 \approx 40$ kHz du signal reçu. Par ailleurs, le design de l'antenne doit tenir compte de sa bande passante $\Delta\omega_a = R_a/L_a$ qu'on souhaite avoir de même ordre de grandeur que la largeur de bande $\Delta\omega$ du signal RF, afin de ne pas perdre d'information tout en limitant le bruit détecté. C'est donc la valeur de l'inductance propre $L_a$ de l'antenne 5 qui va dicter la résistance et la capacité à choisir, en fonction des caractéristiques fréquentielles désirées. L'antenne primaire inductive 5 est une antenne volumique. On pourra par exemple choisir une antenne de géométrie Helmholtz, en selle de cheval, ou toute autre géométrie volumique plus complexe, notamment gradiométrique.

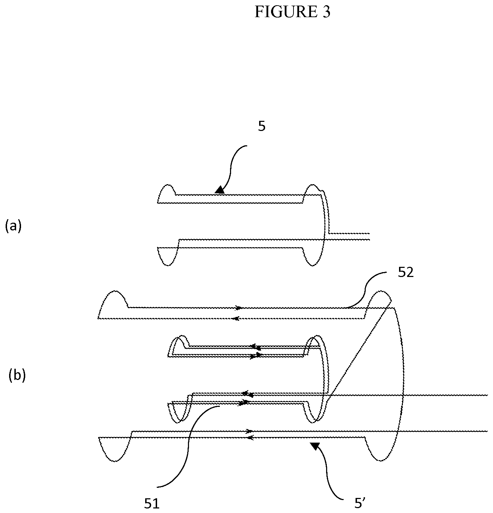

La figure 3 présente deux de ces géométries pour une antenne volumique mise en oeuvre dans un système de détection et d'acquisition RF selon l'invention.

La première géométrie (a) est du type selle de cheval, bien connue de l'homme du métier pour ses performances notamment en termes d'homogénéité spatiale. Le diamètre de l'antenne selle de cheval est égal à 1,5 fois sa longueur.

L'autre géométrie (b) est la version gradiométrique d'ordre 1 de la selle de cheval. Cette antenne volumique 5' est constituée de deux sous-antennes 51,52 montées en série l'une de l'autre. La première antenne 51, interne, est de géométrie selle de cheval et présente dans cet exemple deux tours de fil. La seconde antenne 52, externe et plus grande, également de géométrie selle de cheval, présente un seul tour de fil. Les dimensions du système et l'orientation des fils sont choisis de telle sorte que :

- La partie externe et la partie interne de l'antenne ont la même inductance . Cela est permis par les deux tours de fil dans l'antenne interne.

- Le courant dans la partie interne circule en sens opposé du courant dans la partie externe.

Avec cette configuration, l'antenne gradiométrique-selle de cheval 5' permet de rejeter le bruit provenant de sources se situant à une distance grande devant les dimensions de l'antenne 5', tout en bénéficiant des propriétés d'homogénéité de la géométrie selle de cheval. Une description détaillée du principe des antennes gradiométriques se trouve dans l'article de R. L. Fagaly, « Superconducting quantum interférence device instruments and applications », Review of scientific instruments 77, 101101 (2006).

Concentration de flux et inductance optimale

Pour déterminer l'inductance de l'antenne, on étudie le reste du système de détection en référence à la figure 1. Le dispositif SQUID 3 utilisé (par exemple le modèle SQ680 de StarCryo) est couplé à une bobine d'entrée 8 d'inductance $L_i$ = 720 nH qui réalise le couplage en courant avec l'antenne primaire 5, via un système de transformation de flux 2 matérialisé par les bobines $L_1$ et $L_2$, couplées inductivement. On note $i_1$ (resp. $i_2$) le courant circulant dans l'antenne 5 (resp. la bobine $L_2$) et $\Phi_a$ le flux capté par l'antenne 5. On note par ailleurs $M_{is} = k\sqrt{LiLs}$ l'inductance mutuelle bobine d'entrée-SQUID, et $M_{12} = k^{l}\sqrt{L1L2}$ la mutuelle entre les bobines $L_1$ et $L_2$.

$k$ et $k^l$ sont des facteurs sans dimension et $L_s$ est l'inductance propre du dispositif SQUID 3. On cherche une relation entre le flux extérieur capté par l'antenne, $\Phi_a$, et $\Phi_{sq}$ le flux capté par le SQUID 3.

Les relations de couplage inductif dans le circuit s'écrivent

$$\Phi_a + M_a i_a = (L_a + L_1)i_1 \qquad (1)$$

$$(L_a + L_1)i_1 = M_a i_a \text{ (on néglige l'effet du courant parcourant le SQUID)} \qquad (2)$$

$$\Phi_a = M_a i_a \qquad (3)$$

En combinant ces équations, on obtient

$$\Phi_a = \frac{(L_a + L_1)(L_2 + L_s) - M_{12}^2}{M_a M_{12}} \Phi_s \qquad (4)$$

Cette dernière équation établit un lien entre l'excitation extérieure, donnée par $\Phi_a$, et le niveau de réponse du SQUID 3, quantifié par $\Phi_{sq}$. On comprend alors pourquoi un tel montage est appelé "concentrateur de flux" : le rôle principal de l'antenne Faraday 5 est d'augmenter le flux capté par le SQUID 3.

Le niveau de sensibilité maximale du dispositif, donnant la plus forte réponse en $\Phi_s$ pour un $\Phi_a$ donné, est atteint pour

$$L_1 = \frac{L_a}{\sqrt{1 - k^2}} \qquad (5)$$

$$L_2 = \frac{L_s}{\sqrt{1 - k^2}} \qquad (6)$$

On fixe la résistance de l'antenne primaire 5 à une valeur raisonnable, par exemple $R_a = 1\ \Omega$. Pour respecter la valeur de la bande passante de l'ordre de la dizaine de kH, on doit donc assurer $L_a = 0,1$ mH. Cette valeur de $L_a$ fixe une valeur pour la capacité :

$$C_a = \frac{1}{L_a \omega_0^2} = 6,3 \quad \mu F \qquad (7)$$

Le rapport des inductances $L_1$ et $L_2$ est donc imposé :

$$\frac{L_1}{L_2} = \frac{L_a}{L_s} \qquad (8)$$

soit $L_1$ 1390$L_2$. Les valeurs précises de $L_1$ et de $L_2$ sont fixées par la constante de couplage $k^l$, qu'on cherche à avoir la plus proche possible de 1 afin d'assurer la sensibilité maximale du dispositif.

Nécessité de la transformation de flux

On est en droit de se demander pourquoi on a introduit le couplage inductif via les bobines $L_1$ et $L_2$. Il aurait été

plus simple connecter directement l'antenne avec la bobine d'entrée du SQUID 3. On suppose que le transformateur de flux 2 comprenant les bobines ($L_1$) et ($L_2$) est absent, et que l'antenne inductive d'inductance $L_a$ est en série avec la bobine d'entrée ($L_i$) du SQUID. Le couplage magnétique s'écrit alors

$$\Phi_a = (L_a + L_i)i_1 \tag{9}$$

d'où, en introduisant le couplage au SQUID $\Phi_{sq} = M_{is}i_1$

$$\Phi_a = \frac{L_a + L_i}{k\sqrt{L_i L_s}}\Phi_s \tag{10}$$

L'équation précédente montre que la sensibilité maximale est atteinte pour une inductance de l'antenne 5 en égalisant l'inductance de l'antenne 5 à celle de la bobine d'entrée 8 du SQUID 3: $L_a = L_i$.

On pourra, par exemple, ajuster l'inductance de l'antenne 5 en jouant sur le nombre de tours dans la boucle, ou en jouant sur sa géométrie.

On comprend alors la nécessité d'introduire un transformateur de flux. En effet, sans celui-ci, l'inductance de l'antenne est imposée à la valeur $L_a = L_i$ = 720 nH. Cette valeur d'inductance impose une résistance pour l'antenne

$$R_a = L\Delta\omega = 0,72 \text{ m}\Omega \tag{11}$$

et une capacité à brancher sur l'antenne de valeur

$$C_a = \frac{1}{L\omega_0^2} = 0,87 \quad \text{mF} \tag{12}$$

Ces résultats ne sont pas satisfaisants pour deux raisons. D'une part, la capacité trouvée est extrêmement élevée, à ces valeurs nous devrons utiliser des capacités chimiques qui pourraient ne pas être adaptées au froid du cryostat. D'autre part, la valeur de la résistance est très faible, ce qui aura un impact sur le bruit en intensité dans l'antenne

$$\delta i_a = \sqrt{\frac{4k_B T}{R}} \tag{13}$$

soit, avec une antenne refroidie à 100 K, $\delta i_a \approx 3$ nA/$\sqrt{}$Hz. Ce bruit est beaucoup trop élevé en regard du très faible niveau de bruit en entrée du SQUID de l'ordre du $pA\sqrt{Hz}$.

Une solution est d'augmenter la résistance de l'antenne primaire 5, ce qui nécessite de passer par un transformateur de flux 2 pour adapter l'inductance afin de conserver la même bande passante.

SQUID lecteur de courant

Le dispositif SQUID 3 utilisé est un SQUID bas-Tc (ex : SQ680 de chez STARCryo®) refroidi par *cryocooler* (pour « cryoréfrigérateur », par exemple le PT 403 de CryoMech®, et polarisé par un courant $i_p$.

Contrairement à ses homologues haut-Tc, le SQUID bas-Tc présente un niveau de bruit thermique bien moindre, ce qui permet d'augmenter drastiquement le rapport signal-à-bruit et in fine la qualité de l'image finale. Son rôle est de lire le courant engendré dans la bobine d'entrée, avec un niveau de bruit de 0,8 $pA/\sqrt{}Hz$. Ce niveau de bruit est donc l'objectif à atteindre pour le bruit thermique dans l'antenne inductive.

Amplificateur bas-bruit - FLL

Le dispositif SQUID présente une réponse flux capté-courant non-linéaire, périodique de période le quantum de flux $\Phi_0 = h/2e$. Pour linéariser cette réponse afin d'éviter des artefacts dégradant la qualité d'image, on couple le SQUID 3 à une boucle à verrouillage de flux (FLL), dont un exemple de fonctionnement est décrit ci-après.

Cette boucle présente d'abord un pré-amplificateur 40 (LNA) de la tension mesurée aux bornes du SQUID.

Deux choix sont envisageables pour le système d'amplification: soit opter pour une amplification à SQUID comme c'est par exemple le cas dans le document US2013271142A, ou bien utiliser une amplification à hétérostructure semiconductrices de type ASIC, ce qui est potentiellement plus intéressante mais pose aussi plus de contraintes, notamment dans le niveau maximal d'excursion en amplitude en tension du signal entrant.

La bobine de contre-réaction 10 d'inductance $L_{feed}$ permet de réagir aux variations de flux entrant pour maintenir le SQUID 3 à son niveau de sensibilité en flux maximale. Le signal est luen sortie de la boucle à verrouillage de flux.

Equipement IRM

Un système de détection ultrasensible et d'acquisition RF à base de SQUID peut être intégré dans un équipement d'IRM utilisant une champ magnétique de travail de l'ordre de B0 = 1 mT (ce qui correspond à une fréquence $\omega 0$ 40 kHz), tout en conservant une temps d'acquisition et une qualité d'image en accord avec les standards cliniques actuels. La baisse du champ magnétique de travail de plusieurs ordres de grandeur permet de s'affranchir des contraintes empêchant d'une part une adoption massive de l'IRM comme standard d'imagerie et d'autre part l'ouverture d'applications encore inexistantes telles que l'IRM embarquée dans un camion pour diagnostiquer le type d'AVC (Accident Vasculo-Cérébral) : ischémique ou hémorragique, le dépistage du cancer du sein 100% IRM (effectué au scanner aujourd'hui), ou l'IRM per-opératoire, grâce à un équipement léger, sans blindage magnétique, et peu cher.

La figure 2 présente un schéma de principe d'une expérience d'IRM effectuée avec le système de détection selon l'invention. On a choisi de présenter un IRM du genou, l'imagerie ostéo-articulaire étant un des premiers cas d'application probables de l'invention. Le genou du patient est enfilé dans un cylindre A qui comprend un solénoïde assurant un champ permanent $B_0 \approx 1$ mT homogène à environ 10 ppm sur un volume d'environ 10x10x10 $cm^3$, des gradients, et l'antenne de réception décrite précédemment. L'antenne de réception est refroidie à une température d'environ 60 K à l'aide d'un système de cryogénie sur-mesure dérivé du pulse-tube assurant le refroidissement du système SQUID de la partie B.

La partie B comprend le SQUID assurant la lecture du courant en provenance de l'antenne inductive, ainsi que l'électronique de traitement du signal décrite plus haut le système de pré-amplification, ainsi que la boucle à verrouillage de flux FLL composée d'un amplificateur intégrateur, d'une résistance de lecture, et d'une bobine de bouclage $L_{feed}$. Tout cet étage est refroidi à l'aide d'une machine cryogénique, par exemple le pulse-tube PT403 de CryoMech, à une température avoisinant 4, 2 K.

La partie C assure la conversion analogique-numérique du signal pour un post-traitement par ordinateur afin de piloter l'équipement et d'afficher l'image IRM obtenue.

Applications médicales

La sensibilité et la portabilité du dispositif le rendent intéressant en premier lieu pour l'imagerie à résonance magnétique (IRM). Les hauts niveaux de contraste obtenus à bas champ rendent la technologie intéressante pour les diagnostics ou le contraste est aujourd'hui insuffisant, avec les technologies haut-champ.

Par ailleurs, un équipement selon l'invention pourrait aisément être embarqué dans un camion d'ambulance afin de diagnostiquer rapidement, sur le lieu de l'accident, un AVC ischémique ou hémorragique, afin de prendre en charge plus rapidement le patient et d'éviter des dégâts irrémédiables sur les facultés cognitives.

De par son faible coût et sa facilité d'utilisation, l'équipement d'imagerie selon l'invention pourrait également se répandre largement dans des cas d'usage où il est aujourd'hui trop peu utilisé : dépistage du cancer du sein chez les femmes de plus de 50 ans, utilisation en neurologie et en psychiatrie : dépistage précoce des maladies telles que la schizophrénie, la dépression, ou l'épilepsie ; dépistage du cancer de la prostate.

Enfin, de nombreux projets d'IRM bas champ à base de SQUID ont également pour but la conception d'un appareil hybride IRM - magnétoencéphalographie (MEG). C'est par exemple le cas du travail de l'équipe de l'université d'Aalto en Finlande, en référence à la source https://www.aalto.fi/en/department-of-neuroscience-and-biomedical-engineering/meg-mri-brain-imaging-group.

Un équipement IRM à base de SQUID selon l'invention pourra être adapté pour intégrer en son sein un dispositif de MEG.

Résonance magnétique nucléaire

Les appareils de RMN utilisés notamment pour la caractérisation chimique peuvent également bénéficier de notre système de détection pour concevoir des équipements plus légers et moins coûteux, pour des raisons similaires à celles avancées dans le cadres de l'IRM.

Industrie minière

Il existe déjà dans l'industrie minière des capteurs magnétiques à base de SQUID pour la prospection de métaux, comme l'illustre le document US 7,394,250. Notre système de détection peut également être intégré dans un tel appareil pour la prospection minière, grâce à son très bas niveau de bruit. Le principe est le suivant : on émet une onde RF dans le sol, si une veine métallique est présente, des courants de Foucault sont induits dans la veine qui émet à son tour une onde RF, cette onde est détectée par notre appareil intégrant le système de détection à SQUID.

Domaine militaire

Les capteurs radiofréquence ultrasensibles sont des éléments bien connus des systèmes de guerre électronique : ils servent par exemple à la détection de signaux de communication. Une autre application intéressante est la détection de submersibles immergés : le submersible étant constitué de matériaux ferromagnétiques, notre dispositif est capable de détecter sa présence par l'émission d'ondes RF et la détection des ondes produites par les courants de Foucault induits, sur le même principe que la prospection minière. D'autres systèmes détectent quand à eux la perturbation du champ terrestre local engendré par le passage du sous-marin, comme l'illustre le document « Magnetic détection of a surface ship by an airborne LTS SQUID MAD » par Megumi Hirota et al., April 2001, IEEE Transactions on Applied Superconductivity 11(1):884 - 887.

Radioastronomie

Les systèmes à base de SQUID sont déjà largement utilisés dans le domaine de la radioastronomie, par exemple intégrés à des bolomètres supraconducteurs pour la lecture et/ou l'amplification de courants très faibles, Par sa très haute sensibilité, notre système pourra trouver une intégration intéressante dans un télescope calibré dans le domaine RF.
Bien entendu, la présente invention n'est pas limitée aux modes de réalisation qui viennent d'être décrits et de nombreuses autres variantes de réalisation peuvent être envisagées dans le cadre de l'invention.

**Revendications**

1. Système (1) de détection et d'acquisition radiofréquence (RF) à base de SQUID, prévu notamment pour être intégré dans un appareil (S) à résonance magnétique nucléaire, par exemple un appareil d'imagerie par résonance magnétique (IRM), comprenant:

   - une antenne primaire de détection de type volumique (5),
   - un transformateur de flux (2),
   - un dispositif SQUID (3), agencé pour capter le flux magnétique capturé par l'antenne primaire (5) et reproduit par un enroulement d'entrée (8) au sein dudit dispositif SQUID via le transformateur de flux (2), et délivrer un signal de détection secondaire,
   - un dispositif de cryogénie prévu pour refroidir le dispositif SQUID (3),
   - un étage (4) de traitement du signal de détection secondaire émis par le dispositif SQUID (3), pour délivrer un signal analogique d'acquisition, comprenant une boucle à verrouillage de flux (FLL) prévue pour linéariser la réponse du dispositif SQUID (3).

   **caractérisé en ce que** le dispositif SQUID est de type à basse température critique, la transformateur de flux (2) a un enroulement primaire (6) relié à l'antenne primaire de détection (5), le dispositif de cryogénie est en outre prévu pour refroidir le transformateur de flux, et **en ce que** l'antenne primaire de détection (5) comprend des bobines de Helmholtz ou en selles de cheval..

2. Système de détection et d'acquisition selon la revendication 1 **caractérisé en ce que** l'antenne primaire de détection présente une géométrie gradiométrique.

3. Système de détection et d'acquisition (1) selon la revendication 1, **caractérisé en ce que** la boucle à verrouillage de flux (FLL) comprend un amplificateur bas-bruit (LNA).

4. Système de détection et d'acquisition (1) selon la revendication 3, **caractérisé en ce que** l'amplificateur bas-bruit comprend un amplificateur à hétérostructure semiconductrice.

5. Système de détection et d'acquisition selon l'une des revendications 3 ou 4, **caractérisé en ce que** l'amplificateur bas-bruit comprend un système d'amplification à base de SQUID.

6. Système de détection et d'acquisition selon l'une quelconque des revendications précédentes, **caractérisé en ce**

**qu'**il comprend en outre une ou plusieurs bobines de compensation active du bruit externe audit système.

7. Système de détection et d'acquisition (1) selon l'une quelconque des revendications précédentes, **caractérisé en ce que** l'antenne primaire de détection (5) coopère avec le transformateur de flux (2) pour concentrer le flux capté par le dispositif SQUID.

8. Système de détection et d'acquisition (1) selon l'une quelconque des revendications précédentes, **caractérisé en ce qu'**il comprend en outre au sein du transformateur de flux (2) une bobine de contre-réaction d'inductance (10) agencée pour réagir aux variations du flux entrant, de façon à maintenir le dispositif SQUID (3) à son niveau de sensibilité en flux maximale.

9. Equipement (S) d'imagerie par résonance magnétique (IRM) comprenant :

   - un système (B) de détection et d'acquisition radiofréquence (RF) à base de SQUID selon l'une quelconque des revendications précédentes,
   - un dispositif porte-antenne (A), intégrant l'antenne primaire de détection de type volumique et relié audit système (B) de détection et d'acquisition,
   - un étage de conversion analogique-numérique (C), prévu pour convertir le signal analogique d'acquisition en données numériques adaptées pour un post-traitement en vue de générer et afficher une image IRM.

10. Equipement IRM selon la revendication précédente, **caractérisé en ce qu'**il est couplé à un dispositif de magnétoencéphalographie (MEG).

11. Equipement de résonance magnétique nucléaire (RMN) incluant un système de détection et d'acquisition RF à base de SQUID selon l'une quelconque des revendications 1 à 8.

12. Equipement capteur magnétique à base de SQUID pour la prospection de métaux, incluant un système de détection et d'acquisition RF selon l'une quelconque des revendications 1 à 8, pour détecter une onde radiofréquence (RF) émise par une veine métallique en réponse à une émission d'une onde radiofréquence (RF) dans un sol.

13. Equipement capteur radiofréquence ultrasensible incluant un système de détection et d'acquisition selon l'une quelconque des revendications 1 à 8.

14. Equipement de radioastronomie opérant dans le domaine des radiofréquences (RF) incluant in système de détection et d'acquisition RF à base de SQUID selon l'une quelconque des revendications 1 à 8.

**Patentansprüche**

1. SQUID-basiertes Hochfrequenz-Erkennungs- und Erfassungssystem (HF-Erkennungs- und Erfassungssystem) (1), das insbesondere zum Integriertwerden in eine Kernspinresonanzeinrichtung (S), zum Beispiel eine Magnetresonanztomographieeinrichtung (MRT), vorgesehen ist, umfassend:

   - eine primäre Erkennungsantenne (5) von dem Volumentyp,
   - einen Flusstransformator (2),
   - eine SQUID-Vorrichtung (3), die zum Aufnehmen des magnetischen Flusses, der durch die primäre Antenne (5) aufgenommen und durch eine Eingangswicklung (8) innerhalb der SQUID-Vorrichtung über den Flusstransformator (2) reproduziert wird, und zum Liefern eines sekundären Erkennungssignals angeordnet ist,
   - eine Kryogenikvorrichtung, die zum Kühlen der SQUID-Vorrichtung (3) vorgesehen ist,
   - eine Stufe (4) zum Verarbeiten des sekundären Erkennungssignals, das durch die SQUID-Vorrichtung (3) ausgegeben wird, zum Liefern eines analogen Erfassungssignals, umfassend eine Flussregelschleife (FLL), die zum Linearisieren der Reaktion der SQUID-Vorrichtung (3) vorgesehen ist.
   **dadurch gekennzeichnet, dass** die SQUID-Vorrichtung von dem Typ mit niedriger kritischer Temperatur ist, der Flusstransformator (2) eine primäre Wicklung (6) besitzt, die mit der primären Erkennungsantenne (5) verbunden ist, die Kryogenikvorrichtung ferner zum Kühlen des Flusstransformators vorgesehen ist, und **dass** die primäre Erkennungsantenne (5) Helmholtz- oder Sattelspulen umfasst.

2. Erkennungs- und Erfassungssystem nach Anspruch 1,

**dadurch gekennzeichnet, dass** die primäre Erkennungsantenne eine gradiometrische Geometrie aufweist.

3. Erkennungs- und Erfassungssystem (1) nach Anspruch 1,
**dadurch gekennzeichnet, dass** die Flussregelschleife (FLL) einen rauscharmen Verstärker (LNA) umfasst.

4. Erkennungs- und Erfassungssystem (1) nach Anspruch 3,
**dadurch gekennzeichnet, dass** der rauscharme Verstärker einen Halbleiterheterostrukturverstärker umfasst.

5. Erkennungs- und Erfassungssystem nach einem der Ansprüche 3 oder 4,
**dadurch gekennzeichnet, dass** der rauscharme Verstärker ein SQUID-basiertes Verstärkungssystem umfasst.

6. Erkennungs- und Erfassungssystem nach einem der vorstehenden Ansprüche, **dadurch gekennzeichnet, dass** es ferner eine oder mehrere Spulen für eine aktive Kompensation des Rauschens außerhalb des Systems umfasst.

7. Erkennungs- und Erfassungssystem (1) nach einem der vorstehenden Ansprüche, **dadurch gekennzeichnet, dass** die primäre Erkennungsantenne (5) zum Konzentrieren des Flusses, der durch die SQUID-Vorrichtung aufgenommen wird, mit dem Flusstransformator (2) zusammenwirkt.

8. Erkennungs- und Erfassungssystem (1) nach einem der vorstehenden Ansprüche, **dadurch gekennzeichnet, dass** es ferner innerhalb des Flusstransformators (2) eine induktive Rückkopplungsspule (10) umfasst, die zum Reagieren auf Schwankungen des eingehenden Flusses angeordnet ist, um die SQUID-Vorrichtung (3) bei maximalem Fluss auf seinem Empfindlichkeitsniveau zu halten.

9. Ausrüstung (S) für eine Magnetresonanztomographie (MRT), umfassend:

   - ein SQUID-basiertes Hochfrequenz-Erkennungs- und Erfassungssystem (HF-Erkennungs- und Erfassungssystem) (B) nach einem der vorstehenden Ansprüche,
   - eine antennentragende Vorrichtung (A), die die primäre Erkennungsantenne von dem Volumentyp integriert und mit dem Erkennungs- und Erfassungssystem (B) verbunden ist,
   - eine Analog-Digital-Umwandlungsstufe (C), die zum Umwandeln des analogen Erfassungssignals in digitale Daten vorgesehen ist, die für eine Nachbearbeitung angepasst sind, um ein MRT-Bild zu erzeugen und anzuzeigen.

10. MRT-Ausrüstung nach dem vorstehenden Anspruch,
**dadurch gekennzeichnet, dass** sie mit einer Magnetenzephalographievorrichtung (MEG) gekoppelt ist.

11. Kernspinresonanzausrüstung (NMR), einschließlich eines SQUID-basierten HF-Erkennungs- und Erfassungssystems nach einem der Ansprüche 1 bis 8.

12. Magnetsensorausrüstung auf SQUID-Basis für die Metallsuche, einschließlich eines HF-Erkennungs- und Erfassungssystems nach einem der Ansprüche 1 bis 8, zum Erkennen einer Hochfrequenzwelle (HF-Welle), die durch eine Metallader als Reaktion auf eine Emission einer Hochfrequenzwelle (HF-Welle) in einen Boden emittiert wird.

13. Hochempfindliche Hochfrequenzsensorausrüstung, einschließlich eines Erkennungs- und Erfassungssystems nach einem der Ansprüche 1 bis 8.

14. Funkastronomieausrüstung, die in dem Bereich der Hochfrequenzen (HF) arbeitet, einschließlich eines SQUID-basierten HF-Erkennungs- und Erfassungssystems nach einem der Ansprüche 1 bis 8.

**Claims**

1. System (1) for radio frequency (RF) detection and acquisition based on SQUID, provided in particular to be integrated into a nuclear magnetic resonance apparatus (S), for example a magnetic resonance imaging (MRI) apparatus, comprising:

   - a volume-type primary detection antenna (5),
   - a flux transformer (2),

- a SQUID device (3), arranged to capture the magnetic flux captured by the primary antenna (5) and reproduced by an input winding (8) within said SQUID device via the flux transformer (2), and to deliver a secondary detection signal,
- a cryogenic device designed to cool the SQUID device (3),
- a stage (4) of processing the secondary detection signal emitted by the SQUID device (3) to deliver an analog acquisition signal, comprising a flux-locked loop (FLL) provided to linearize the response of the SQUID device (3), **characterized in that** the SQUID device is a low critical temperature device, the flux transformer (2) has a primary winding (6) connected to the primary detection antenna (5), the cryogenic device is further provided for cooling the flux transformer, **and in that** the primary detection antenna (5) comprises Helmholtz or saddle coils.

2. Detection and acquisition system according to claim 1,
   **characterized in that** the primary detection antenna has a gradiometric geometry.

3. Detection and acquisition system (1) according to claim 1,
   **characterized in that** the flux-locked loop (FLL) comprises a low-noise amplifier (LNA).

4. Detection and acquisition system (1) according to claim 3,
   **characterized in that** the low-noise amplifier comprises a semiconductor heterostructure amplifier.

5. Detection and acquisition system according to one of claims 3 or 4,
   **characterized in that** the low-noise amplifier comprises a SQUID-based amplification system.

6. Detection and acquisition system according to any of the preceding claims, **characterized in that** it further comprises one or more active noise compensation coils for noise external to said system.

7. Detection and acquisition system (1) according to any of the preceding claims, **characterized in that** the primary detection antenna (5) cooperates with the flux transformer (2) to concentrate the flux captured by the SQUID device.

8. Detection and acquisition system (1) according to any of the preceding claims, **characterized in that** it further comprises within the flux transformer (2) an inductance feedback coil (10) arranged to react to the variations of the incoming flux, so as to keep the SQUID device (3) at its maximum flux sensitivity level.

9. Magnetic resonance imaging (MRI) equipment item (S) comprising:

   - a SQUID-based radio frequency (RF) detection and acquisition system (B) according to any of the preceding claims,
   - an antenna holder device (A), integrating the volume primary detection antenna and connected to said detection and acquisition system (B),
   - an analog-to-digital conversion stage (C), designed to convert the analog acquisition signal into digital data suitable for post-processing to generate and display an MRI image.

10. MRI equipment item according to the preceding claim,
    **characterized in that** it is coupled to a magnetoencephalography (MEG) device.

11. Nuclear magnetic resonance (NMR) equipment item including a SQUID-based RF detection and acquisition system according to any of claims 1 to 8.

12. SQUID-based magnetic sensor equipment item for the prospecting of metals, including an RF detection and acquisition system according to any of claims 1 to 8, for detecting a radio frequency (RF) wave emitted by a metal vein in response to an emission of a radio frequency (RF) wave in a ground.

13. Ultra-sensitive radio frequency sensor equipment item including a detection and acquisition system according to any of claims 1 to 8.

14. Radio astronomy equipment item operating in the radio frequency (RF) domain including a SQUID-based RF detection and acquisition system according to any of claims 1 to 8.

FIGURE 1

FIGURE 2

FIGURE 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 105137374 A
- JP 2010256318 A
- CN 1287160 C
- WO 2006052236 A1

- US 2013271142 A1
- US 20120206136 A1
- US 2013271142 A
- US 7394250 B

**Littérature non-brevet citée dans la description**

- : ''A compact SQUID-detected magnetic resonance imaging system under microtesla field in a magnetically unshielded environment''. **CHEN HSIN-HSIEN et al.** Journal of Applied Physics. American Institute of Physics, 01 November 2011, vol. 110
- **DE S.K. LEE et al.** SQUID-detected MRI at 132 $\mu$T with Ti-weighted contrast established at 10 $\mu$T-300 mT. *Journal « Magnetic Résonance in Medicine*, January 2005, vol. 53-1, 9-14

- **JOHN CLARKE**. The SQUID handbook : Fundamentals of Technology and Applications of SQUIDs and SQUID systems. Wiley-VCH, 2004
- **R. L. FAGALY**. Superconducting quantum interférence device instruments and applications. *Review of scientific instruments*, 2006, vol. 77, 101101
- **MEGUMI HIROTA et al.** Magnetic détection of a surface ship by an airborne LTS SQUID MAD. *IEEE Transactions on Applied Superconductivity*, April 2001, vol. 11 (1), 884-887